# EUROPEAN PATENT SPECIFICATION

(11) **EP 4 212 125 B1**
(45) Date of publication and mention of the grant of the patent: **11.03.2026**
(21) Application number: 21870773.5
(22) Date of filing: 25.04.2021
(51) Int. Cl.: A61B 90/20, A61B 90/25, G02B 7/00, G02B 21/00, G02B 21/22, A61B 90/00, A61B 90/30

(54) **OPERATING MICROSCOPE FOR TWO SURGEONS**
OPERATIONSMIKROSKOP FÜR ZWEI CHIRURGEN
MICROSCOPE OPÉRATOIRE POUR DEUX CHIRURGIENS

(30) Priority: 23.09.2020 CN 202011009431
(43) Date of publication of application: 19.07.2023
(73) Proprietor: Zumax Medical Co., Ltd., Jiangsu 215129 (CN)
(72) Inventor: WANG, Jilong, Suzhou, Jiangsu 215129 (CN); HE, Jin, Suzhou, Jiangsu 215129 (CN); LI, Jianyue, Suzhou, Jiangsu 215129 (CN); DU, Lei, Suzhou, Jiangsu 215129 (CN); ZHU, Chengjie, Suzhou, Jiangsu 215129 (CN)
(74) Representative: Sun, Yiming
(86) International application number: PCT/CN2021/089575
(87) International publication number: WO 2022/062384

(56) References cited:
- CN-A- 102 495 463
- CN-A- 109 745 124
- CN-A- 112 190 348
- CN-U- 202 631 839
- CN-U- 203 263 352
- CN-U- 205 433 937
- JP-A- 2006 050 320
- JP-A- H05 107 482
- US-A- 5 867 210
- US-A1- 2003 053 202
- US-A1- 2006 018 017
- US-A1- 2014 247 482
- US-A1- 2019 282 063

## Description

### FIELD OF THE INVENTION

The present disclosure relates to the technical field of medical equipment, and in particular to a surgical microscope having assistant's device.

### BACKGROUND TECHNOLOGY

An exit pupil position of an eyepiece of a traditional surgical microscope is fixed, and an exit pupil diameter is generally only about 2 mm. In order to observe the complete field of view of an object plane, an operator needs to keep the eye pupils at the exit pupil position of the eyepiece for a long time. Even if the microscope is ergonomically designed, the operator may easily feel tired by keeping the posture unchanged for a long time. For some special affected parts, the surgical microscope needs to tilt to a great extent for observation. At this moment, the operator also needs to follow the eyepiece to adjust his/her position. Although some surgical microscopes are provided with compensation structures, most of the compensation ranges are limited, and manual adjustment is needed every time, so that the operation is complicated and inconvenient.

Based on the above reasons, in some technical solutions, a display is used for displaying video images, but the ordinary display cannot show depth information of an object, and it is not applicable to a real-time operation.

In some other technical solutions, a 3D display based on a polarization principle is used, an observer can only see a stereoscopic image by wearing polarized glasses, and it is unfriendly for a glass-wearing operator. In addition, the observer can only observe an ideal stereoscopic image at a posture of almost right facing the display. Moreover, the size of the display adopting this solution is generally large, so a distance from a placing position of the display to the operator is generally 2 m or above. Since there is an adjusting process when the human eyes observe objects with great difference in distance, when the operator moves his/her sight from the display to observe or adjust microscope parameters or other auxiliary equipment and then returns to the display, he/she cannot immediately see detail images on the display clearly.

Particularly for complicated operations, the operator generally needs an assistant for microscopical synchronous observation and cooperated operation. Since the positions of the assistant and the operator relative to a patient are different, the posture of at least one person cannot keep comfort direct viewing during the observation on the same display. Moreover, due to the difference of observation angles, the sense of direction of the assistant may be confused when the assistant and the operator observe the same display.

Particularly, in many clinical departments, particularly in the orthopedics department, the operator and the assistant need to operate in a face-to-face manner. At this moment, according to an existing solution, a bridge type light splitting device (US20030133187) with a cumbersome and complicated structure is used, of which an inside optical path system has multiple transitions and at least one intermediate imaging, and the luminous flux loss is serious, so that the observation resolution ratio and the contrast ratio are reduced. In addition, the system is complicated, and the manufacturing cost is high.

A medical image processing apparatus including an image processing section including a function of processing a medical image of an observation target captured by an imaging device in correspondence with a target display apparatus is disclosed in US 2019/0282063 A1.

Therefore, in combination with the technical problems mentioned above, it is necessary to provide a new technical solution.

### SUMMARY OF THE INVENTION

The purpose of the present disclosure is to provide a surgical microscope having assistant's device, which enables an operator and an assistant of different body sizes to maintain reasonable ergonomic postures, see true images of their respective perspectives, observe the state of each other at any time and communicate in facial language. Meanwhile, only one set of stereo optical imaging system is needed, and two persons can observe at the same time by simply assigning and flipping video signals.

In order to achieve the purpose of the present disclosure, according to one aspect of the present disclosure, the present disclosure provides a surgical microscope as defined in claim 1. The microscope having assistant's device, which comprises a support, a microscope body, a first naked eye 3D display and a second naked eye 3D display. The microscope body is mounted on the support, a photosensitive element is arranged in the microscope body, the first naked eye 3D display and the second naked eye 3D display are respectively connected to the photosensitive element, display directions of the first naked eye 3D display and the second naked eye 3D display are opposite, and directions of an image displayed by the first naked eye 3D display and an image displayed by the second naked eye 3D display are different by 180 degrees.

In a further embodiment, the surgical microscope having assistant's device further comprises a mounting rack, and the first naked eye 3D display and the second naked eye 3D display are respectively movably mounted on the mounting rack.

In a further embodiment, the first naked eye 3D display and the second naked eye 3D display are respectively able to be driven to independently move on the mounting rack in a vertical direction.

In a further embodiment, the support comprises a base, a support rod vertically mounted on the base, a large cross arm rotatably mounted on the support rod, a small cross arm rotatably mounted on the large cross arm and a balance arm rotatably mounted on the small cross arm, and the microscope body is mounted on the balance arm.

In a further embodiment, the first naked eye 3D display and the second naked eye 3D display are mounted on the microscope body, the large cross arm or the support rod through the mounting rack; or the surgical microscope having assistant's device further comprises a seat body and a connecting rod mounted on the seat body, and the first naked eye 3D display and the second naked eye 3D display are mounted on one end of the connecting rod through the mounting rack.

In a further embodiment, the other end of the connecting rod is movably mounted on the seat body, the connecting rod is able to be driven to move in an axis direction, and/or the connecting rod is able to be driven to rotate by taking an axis of the connecting rod as a rotating shaft.

In a further embodiment, the first naked eye 3D display and the second naked eye 3D display are able to be placed on the ground or suspended on a roof through the seat body and the connecting rod.

In a further embodiment, a size of the first naked eye 3D display and the second naked eye 3D display is between 12 inches and 16 inches; and the microsurgery auxiliary device further comprises an acquisition device, a processing device and a driving device, the acquisition device is able to be configured to acquire eye position information of an operator or an assistant, and the processing device is configured to be able to control the driving device to act according to the acquired eye position information to regulate a display angle of the first naked eye 3D display and the second naked eye 3D display.

In a further embodiment, the surgical microscope having assistant's device further comprises a plurality of wheels, and the wheels are mounted at a bottom end of the base or the seat body.

In a further embodiment, the wheels are universal wheels.

### BRIEF DESCRIPTION OF THE DRAWINGS

FIG. 1 is a schematic structural diagram of a surgical microscope having assistant's device provided by one embodiment of the present application.
FIG. 2 is a schematic connection diagram of two naked eye 3D displays of a surgical microscope having assistant's device provided by one embodiment of the present application.
FIG. 3 is a three-dimensional schematic mounting diagram of two naked eye 3D displays of a surgical microscope having assistant's device provided by one embodiment of the present application when being placed on the ground.
FIG. 4 is a lateral-view schematic mounting diagram of two naked eye 3D displays of a surgical microscope having assistant's device provided by one embodiment of the present application when being placed on the ground.
FIG. 5 is a schematic mounting diagram of two naked eye 3D displays of a surgical microscope having assistant's device provided by one embodiment of the present application when being suspended on a roof.
FIG. 6 is a schematic structural diagram of a surgical microscope having assistant's device provided by one embodiment of the present application.
FIG. 7a and FIG. 7b are schematic diagrams of optical path principles of a surgical microscope having assistant's device provided by one embodiment of the present application in two states when adjusting a distance between a plus lens set and a minus lens set.
FIG. 8 is a schematic diagram of an optical path principle of a surgical microscope having assistant's device provided by one embodiment of the present application under a condition of being provided with an observation unit.
FIG. 9 is a schematic structural diagram of a surgical microscope having assistant's device provided by one embodiment of the present application under a condition of being provided with an observation unit.
FIG. 10a and FIG. 10b are schematic diagrams of optical path principles of a surgical microscope having assistant's device provided by one embodiment of the present application in two states under a condition of being provided with double illumination optical paths.
FIG. 11 is a schematic diagram of an optical path principle of a surgical microscope having assistant's device provided by one embodiment of the present application when a projection microscope set and a zooming microscope set are linked.
FIG. 12 is a schematic diagram of viewing angle and distance of naked eye 3D displays of a surgical microscope having assistant's device provided by one embodiment of the present application.
FIG. 13a to FIG. 13c are schematic structural diagrams of a surgical microscope having assistant's device provided by one embodiment of the present application when naked eye 3D displays are mounted at other positions of a support.

In the figures, 1 denotes support, 11 denotes base, 12 denotes large cross arm, 13 denotes small cross arm, 14 denotes balance arm, 15 denotes support rod, 2 denotes microscope body, 20 denotes imaging unit, 21 denotes large objective set, 211 denotes plus lens set, 212 denotes minus lens set, 2121 denotes outer side surface, 2122 denotes inner side surface, 22 denotes zooming microscope set, 221 denotes second lens, 23 denotes first lens cone objective, 24 denotes photosensitive element, 25 denotes observation optical path, 26 denotes spectroscope set, 3 denotes first naked eye 3D display, 4 denotes second naked eye 3D display, 5 denotes mounting rack, 6 denotes seat body, 61 denotes connecting rod, 62 denotes wheel, 7 denotes illumination unit, 71 denotes light source assembly, 711 denotes LED light source, 72 denotes collecting lens set, 73 denotes diaphragm, 74 denotes projection microscope set, 741 denotes first lens, 75 denotes illumination optical path, 8 denotes observation unit, 81 denotes eyepiece, 82 denotes deflecting prism set, 83 denotes second lens cone objective, 9 denotes operator, and 10 denotes assistant.

### DETAILED DESCRIPTION OF THE PREFERRED

### EMBODIMENTS

In order to further illustrate the technical means and effects adopted by the present disclosure to achieve the intended purpose of the present disclosure, a detailed description of the specific implementations, structures, features and effects according to the present disclosure will be given below with reference to the accompanying drawings and exemplary embodiments.

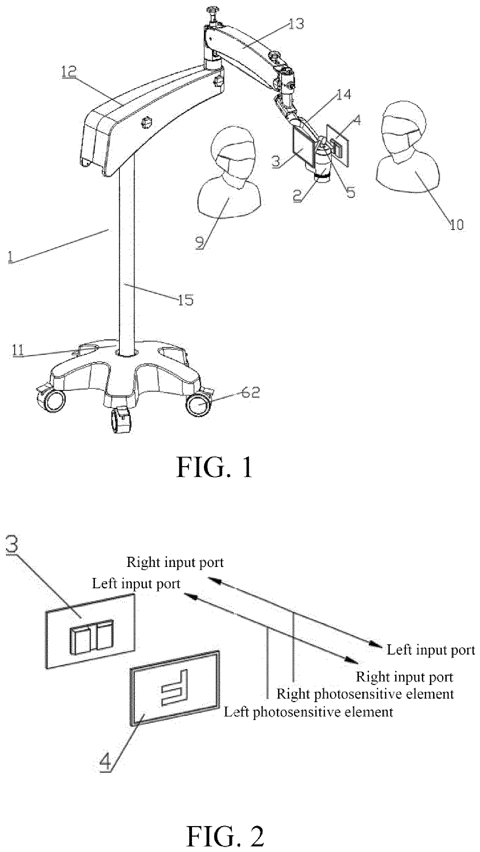

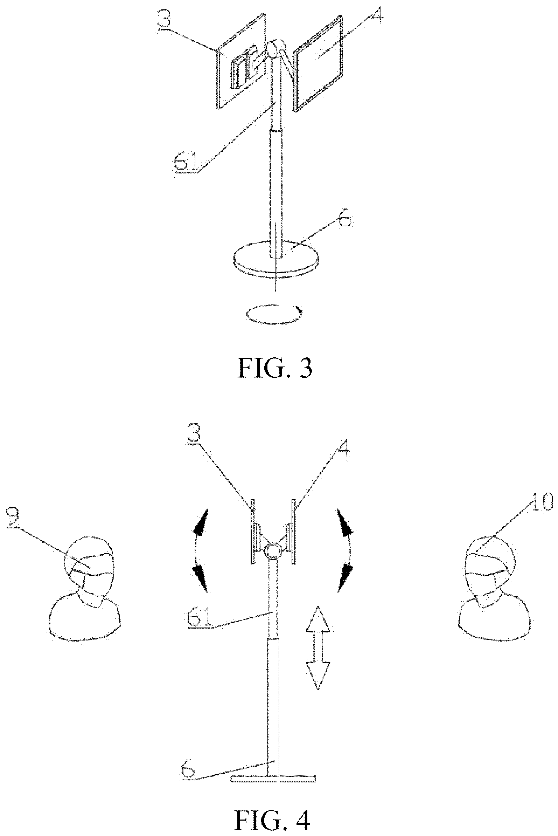

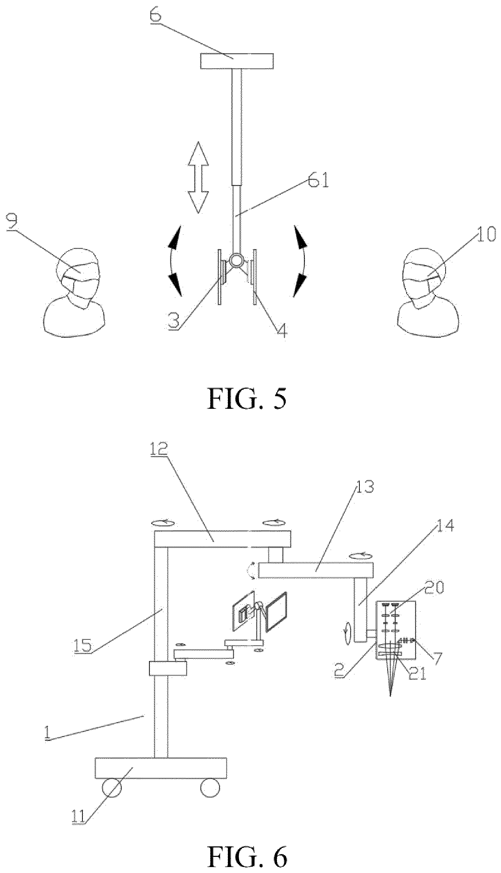

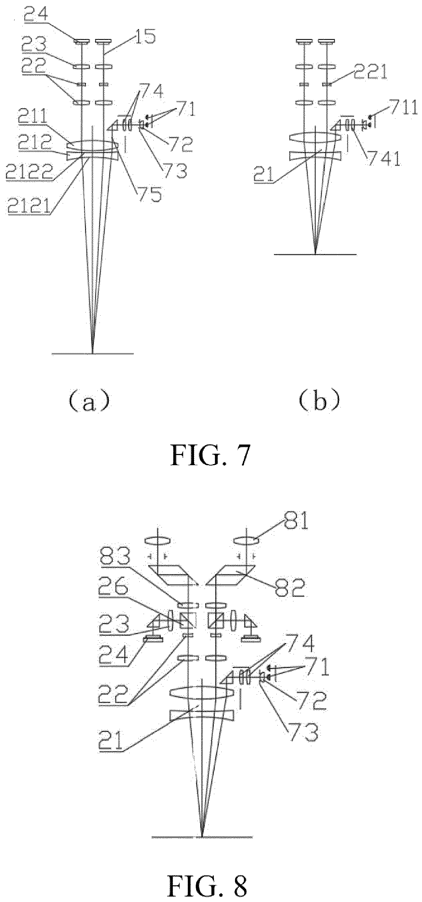

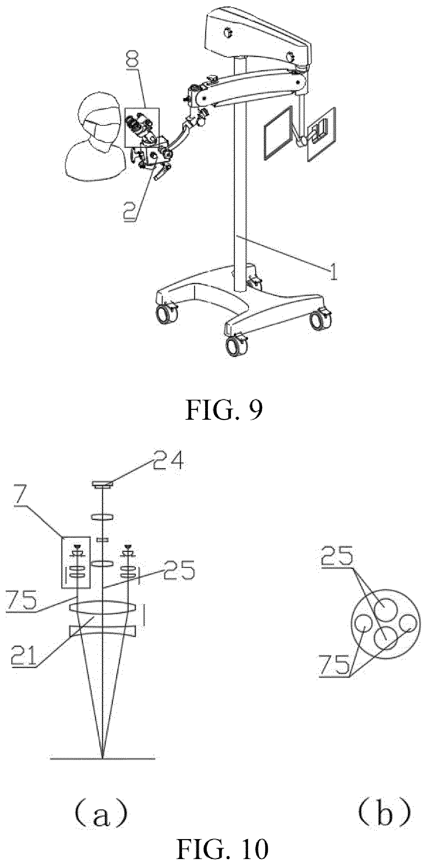

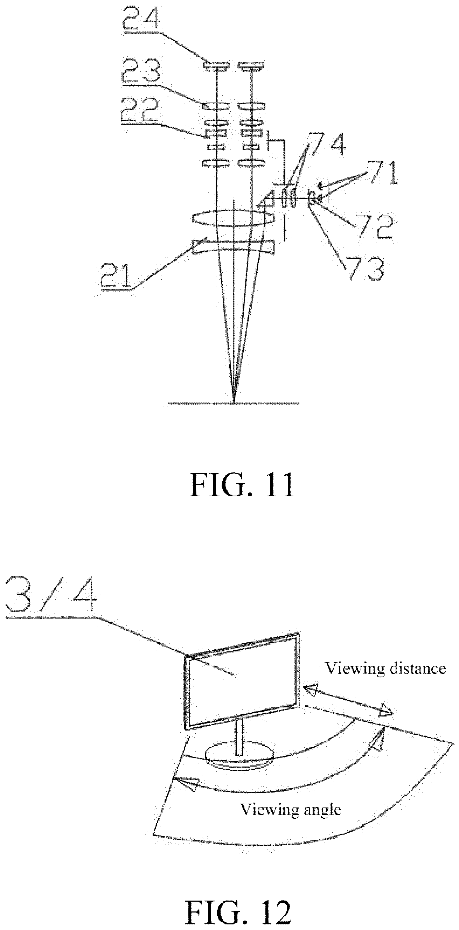

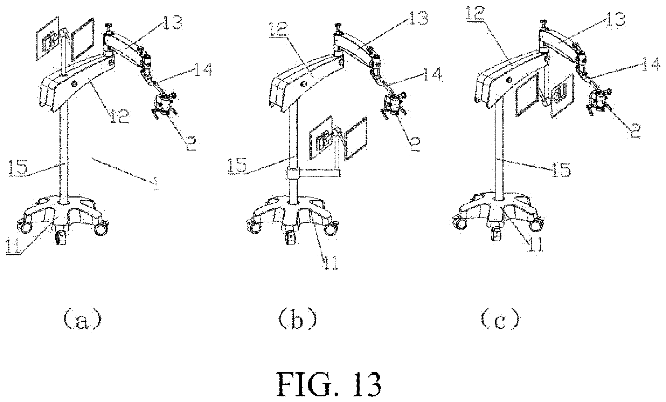

Referring to FIG. 1 to FIG. 13, FIG. 1 is a schematic structural diagram of a surgical microscope having assistant's device provided by one embodiment of the present application; FIG. 2 is a schematic connection diagram of two naked eye 3D displays of a surgical microscope having assistant's device provided by one embodiment of the present application; FIG. 3 is a three-dimensional schematic mounting diagram of two naked eye 3D displays of a surgical microscope having assistant's device provided by one embodiment of the present application when being placed on the ground; FIG. 4 is a lateral-view schematic mounting diagram of two naked eye 3D displays of a surgical microscope having assistant's device provided by one embodiment of the present application when being placed on the ground; FIG. 5 is a schematic mounting diagram of two naked eye 3D displays of a surgical microscope having assistant's device provided by one embodiment of the present application when being suspended on a roof; FIG. 6 is a schematic structural diagram of a surgical microscope having assistant's device provided by one embodiment of the present application; FIG. 7a and FIG. 7b are schematic diagrams of optical path principles of a surgical microscope having assistant's device provided by one embodiment of the present application in two states when adjusting a distance between a plus lens set and a minus lens set; FIG. 8 is a schematic diagram of an optical path principle of a surgical microscope having assistant's device provided by one embodiment of the present application under a condition of being provided with an observation unit; FIG. 9 is a schematic structural diagram of a surgical microscope having assistant's device provided by one embodiment of the present application under a condition of being provided with an observation unit; FIG. 10a and FIG. 10b are schematic diagrams of optical path principles of a surgical microscope having assistant's device provided by one embodiment of the present application in two states under a condition of being provided with double illumination optical paths; FIG. 11 is a schematic diagram of an optical path principle of a surgical microscope having assistant's device provided by one embodiment of the present application when a projection microscope set and a zooming microscope set are linked; FIG. 12 is a schematic diagram of viewing angle and distance of naked eye 3D displays of a surgical microscope having assistant's device provided by one embodiment of the present application; and FIG. 13a to FIG. 13c are schematic structural diagrams of a surgical microscope having assistant's device provided by one embodiment of the present application when naked eye 3D displays are mounted at other positions of a support.

### Embodiments

As shown in FIG. 1, a surgical microscope having assistant's device according to this embodiment comprises a support 1, a microscope body 2, a first naked eye 3D display 3 and a second naked eye 3D display 4. The support 1 comprises a base 11, a support rod 15 vertically mounted on the base 11, a large cross arm 12 rotatably mounted on the support rod 15, a small cross arm 13 rotatably mounted on the large cross arm 12 and a balance arm 14 rotatably mounted on the small cross arm 13, and the microscope body 2 is mounted on the balance arm 14, as shown in FIG. 1, FIG. 6 or FIGs. 13a-c. An imaging unit 20 is arranged in the microscope body 2. The imaging unit 20 comprises a large objective set 21, a zooming microscope set 22, a first lens cone objective 23 and a photosensitive element 24, and the large objective set 21, the zooming microscope set 22, the first lens cone objective 23 and the photosensitive element 24 are sequentially located in a same observation optical path 25, as shown in FIG. 6.

The large objective set 21 comprises at least one plus lens set 211 and at least one minus lens set 212. The plus lens set 211 and the minus lens set 212 are arranged in a manner of sharing a same optical axis, a distance between the plus lens set 211 and the minus lens set 212 is adjustable, and an adjusting range of the distance between the plus lens set 211 and the minus lens set 212 is not less than 6 mm. A focal plane position, i.e., an operating work distance can be conveniently and fast changed by the large objective with variable focal length, so as to cover a required operation depth. A realization manner is to change the distance between the plus lens set 211 and the minus lens set 212, and an adjusting range of the work distance is in direct proportion to a distance range between the plus lens set 211 and the minus lens set 212, as shown in FIG. 7a and FIG. 7b. The plus lens set 211 comprises optical lenses made of at least two different materials. The minus lens set 212 comprises optical lenses made of at least two different materials. The minus lens set 212 is close to an object to be observed. The minus lens set 212 comprises an outer side surface 2121 and an inner side surface 2122, both the outer side surface 2121 and the inner side surface 2122 are concave surfaces, and the absolute value of the curvature radius of the outer side surface 2121 is smaller than the absolute value of the curvature radius of the inner side surface 2122.

The present application preferably adopts a design of binocular observation optical paths 25. A zooming microscope set 22, a first lens cone objective 23 and a photosensitive element 24 are respectively arranged in each of the observation optical paths 25, and the two observation optical paths 25 share one large objective set 21. The double-optical-path zooming microscope set 22 realizes the observation of different magnifying power, and can achieve the overall and local observation on an affected part. The zooming microscope set 22 is preferably of an afocal Galileo structure, and is able to realize step-by-step zooming or continuous zooming. When the zooming microscope set 22 is of a continuous zooming structure, it comprises at least two groups of second lenses 221, and the second lenses 221 may be driven to move along respective optical axes. Through the combination of the zooming microscope set 22 and the large objective with variable focal length, the surgical microscope provided by the present application may be used for realizing the convenient and fast observation at different magnifying power on tissues structures at different depths.

The first naked eye 3D display 3 and the second naked eye 3D display 4 are respectively connected to the photosensitive element 24. The size of the first naked eye 3D display 3 and the second naked eye 3D display 4 is between 12 inches and 16 inches. As shown in FIG. 12, a viewing distance between the naked eye 3D display and the observer is between 400 mm and 1200 mm, a viewing angle range of the naked eye 3D display is not smaller than 120 degrees, and preferably, an observation angle is not smaller than 90 degrees. The first naked eye 3D display 3 and the second naked eye 3D display 4 are mounted on one mounting rack 5 in a back-to-back manner. That is, display directions of the two naked eye 3D displays are opposite, as shown in FIG. 1. During use, the two naked eye 3D displays are located between the two observers, such as an operator 9 and an assistant 10. The first naked eye 3D display 3 and the second naked eye 3D display 4 are respectively able to be driven to independently move on the mounting rack 5 in a vertical direction, the operator 9 and the assistant 10 may separately adjust the height of the respective naked eye 3D displays to realize the adaption to different body heights and ensure the best observation height. Meanwhile, the first naked eye 3D display 3 and the second naked eye 3D display 4 are respectively able to be driven to rotate in a horizontal direction or a vertical direction, i.e., the left and right view angle or pitching view angle of the displays are adjusted to achieve the best observation view angle. The operator 9 and the assistant 10 are respectively located at two sides of the object to be observed, and the operator 9 and the assistant 10 have different observation view angles on the object to be observed, so the directions of observed images of the object to be observed are different. In addition, the operator 9 and the assistant 10 are respectively at two sides of the surgical microscope, so the directions of images of the object to be observed seen by the two persons are different by 180 degrees. Therefore, during design, the directions of an image displayed by the first naked eye 3D display 3 and an image displayed by the second naked eye 3D display 4 of the present application are also different by 180 degrees. For example, the object to be observed is an "F" figure, supposed that the first naked eye 3D display 3 displays an "F" image, then an image displayed in the second naked eye 3D display 4 is an inverse "F" image as shown in FIG. 2 by rotating the "F" image by 180 degrees. A general surgical microscope adopts a design of double observation optical paths 25, so a photosensitive device in the microscope body 2 may be provided with two photosensitive elements 24, one observation optical path 25 corresponds to one photosensitive element 24, the two photosensitive elements are respectively defined as a left photosensitive element and a right photosensitive element for performing video signal transmission for the naked eye 3D displays at the same time. Therefore, in order to achieve an effect that the directions of images displayed by the two naked eye 3D displays are different by 180 degrees, input ports of one naked eye 3D display, such as the second naked eye 3D display 4 may be reversely connected, i.e., a left input port of the second naked eye 3D display 4 is connected with the right photosensitive element, a right input port is connected with the left photosensitive element, then, the image in the second naked eye 3D display 4 is vertically reversed by 180 degrees, and an effect of showing an image in a direction being different by 180 degrees from that in the first naked eye 3D display 3 in the second naked eye 3D display 4 is achieved, as shown in FIG. 2. Of course, the purpose may also be achieved in other manners. For example, the second naked eye 3D display 4 is directly rotated for 180 degrees and then mounted, or the image rotation of the second naked eye 3D display 4 is realized in a manner of software.

Different fixing manners may be selected for the first naked eye 3D display 3 and the second naked eye 3D display 4 according to different place conditions and use habits, and fixing structures are simple and reliable. For example, through the mounting rack 5, the first naked eye 3D display 3 and the second naked eye 3D display 4 may be mounted on an upper surface of the large cross arm 12, and is located above the support rod 15, as shown in FIG. 13a; may also be suspended on a lower surface of the large cross arm 12, as shown in FIG. 13c; and may also be directly mounted on the support rod 15, as shown in FIG. 13b. At the same time, the two naked eye 3D displays may be not mounted on the support 1 of an assist device, and may be placed on the ground through a seat body 6 and a connecting rod 61, as shown in FIG. 4, or suspended on a roof, as shown in FIG. 5. The first naked eye 3D display 3 and the second naked eye 3D display 4 are mounted on one end of the connecting rod 61 through the mounting rack 5, and the other end of the connecting rod 61 is movably mounted on the seat body 6. The connecting rod 61 is able to be driven to move relative to the seat body 6 in an axis direction or is able to be driven to rotate by taking an axis of the connecting rod as a rotating shaft, as shown in FIG. 3 to FIG. 5, so that the mounting positions of the two naked eye 3D displays may be adjusted. It needs to be known that regardless of mounting positions of the first naked eye 3D display 3 and the second naked eye 3D display 4, the mounting height, the rotating angle and the overturning angle of the first naked eye 3D display 3 and the second naked eye 3D display 4 are adjustable, and the states may be automatically maintained after the adjustment.

The naked eye 3D displays are arranged in a range of 400 mm to 1200 mm, which is similar to an observation distance of common clinical equipment. When the observer switches the sight between the observation display and other equipment, the eyes do not need repeated focusing, and the time and the labor are saved. The brightness loss is avoided, and the visual fatigue is reduced. Meanwhile, a short observation distance conforms to an approaching habit of eyes during detail distinguishment. The present application uses the naked eye 3D displays, so that the operator 9 or the assistant 10 may directly perform operating operation by observing the naked eye 3D displays. The overall structure of the device is simple. Complicated data processing on the image is not necessary, and the system delay is small. In addition, due to the use of the naked eye 3D displays, the observation angle of the operator 9 or the assistant 10 does not need to be a directly facing angle, the object to be observed can be clearly observed within a certain observation angle range, and the facing direction of the display does not need to be adjusted.

In a further embodiment, the microsurgery auxiliary device further comprises an acquisition device, a processing device and a driving device. The acquisition device is able to be configured to acquire eye position information of the operator 9 or the assistant 10, and the processing device is configured to be able to control the driving device to act according to the acquired eye position information to regulate the display angle of the first naked eye 3D display 3 or the second naked eye 3D display 4, so that an effect that the naked eye 3D displays automatically track the eyes of the operator 9 or the observer and accordingly rotate is achieved to ensure the best observation angle.

At least one illumination unit 7 is also arranged in the microscope body 2, the object to be observed can be illuminated by illumination light rays of each of the illumination units 7 through the large objective set 21, and in addition, the direction of the illumination light rays entering the large objective set 21 is parallel to the direction of an optical axis of the large objective set 21, so that the reflection loss may be reduced, as shown in FIG. 7a. In addition, symmetrical double illumination optical paths 75 may be adopted to enhance the illumination intensity, and the transverse volume of the system is reduced, so that the microscope body 2 can conveniently realize balance, as shown in FIG. 10a and FIG. 10b. The illumination unit 7 comprises a light source assembly 71, a collecting lens set 72, a diaphragm 73 and a projection microscope set 74 which are sequentially positioned in the same illumination optical path 75. The light source assembly 71 comprises at least one LED light source 711, and at least one LED light source 711 in the light source assembly 71 is able to be driven to be switched to the illumination optical path 75 to illuminate the object to be observed. For example, in addition to a white light source, the light source assembly 71 further comprises at least one monochromatic light source (for a fluorescence mode) which can be switched with the white light source to enter the illumination light path 75. The projection microscope set 74 comprises at least one first lens 741, and the first lens 741 is able to be driven to move along its optical axis.

In a further embodiment, the surgical microscope having assistant's device according to the present application may also be provided with a transmission device between the projection microscope set 74 and the zooming microscope set 22, so as to realize the linkage of the projection microscope set 74 and the zooming microscope set 22. As shown in FIG. 11, the transmission device is not directly shown in the figure, and the linkage relationship between the projection microscope set 74 and the zooming microscope set 22 is illustratively shown through a broken line. During low magnifying power observation, the view field diameter of object surface imaging is larger. At this moment, an illumination optical spot needs to cover the whole object surface view field. However, when the low magnifying power is switched to a high magnifying power, the view field diameter of object surface imaging is reduced rapidly. At this moment, the projection microscope set 74 of the illumination optical path 75 is correspondingly adjusted, so that the illumination optical spot may be accordingly reduced. The optical damage risk possibly caused on tissues beyond the view field is reduced. At the same time, the illuminance inside the view field is favorably improved, and the eye subjective luminance reduction during high magnifying power observation is compensated.

In a further embodiment, in necessary, the surgical microscope having assistant's device according to the present application may be provided with an observation unit 8 on the microscope body 2 to realize the traditional visual observation by eyes, as shown in FIG. 9. As shown in FIG. 8, the observation unit 8 comprises an eyepiece 81, a deflecting prism set 82 (or a prism set) and a second lens cone objective 83. The imaging unit 20 further comprises a spectroscope set 26. In the same observation optical path 25, light rays sequentially pass through the large objective set 21 and the zooming microscope set 22 to reach the spectroscope set 26. The spectroscope set 26 split the light rays into two parts. One part of the light rays sequentially pass through the first lens cone objective 23 to reach the photosensitive element 24, and the other part of the light rays sequentially pass through the second lens cone objective 83, the deflecting prism set 82 and the eyepiece 81. Therefore, during use, the two observers may observe the object to be observed through the naked eye 3D displays, and may also observe the object to be observed in a traditional visual observation manner by eyes, which greatly enhances the operability and adaptability of the microsurgery auxiliary device.

In a further embodiment, a bottom end of the base 11 or a bottom end of the seat body 6 may also be provided with a plurality of wheels 62, preferably universal wheels, so that the operator can conveniently move the microscope.

As used herein, the terms "comprise", "comprise", or any other variation thereof, are intended to cover a non-exclusive inclusion, comprise the elements listed, and may also comprise other elements not expressly listed.

The position terms, such as "front", "rear", "upper" and "lower" used herein are defined with reference to the positions of components in the figures and the positions of the components relative to each other, and are only for the sake of clarity and convenience in expressing the technical solution. It should be understood that the use of the position terms is not intended to limit the scope of the present application.

The embodiments described herein and the features in the embodiments can be combined with each other in a case that there is no conflict.

The foregoing descriptions are merely exemplary embodiment of the present disclosure, but are not intended to limit the present disclosure. The scope of the invention is defined by the appended claims.

## Claims

1. A surgical microscope having assistant's device, comprising a support (1), a microscope body (2), a first naked eye 3D display (3) and a second naked eye 3D display (4), wherein the microscope body (2) is mounted on the support (1), two photosensitive elements (24) are arranged in the microscope body (2), wherein the two photosensitive elements (24) are respectively defined as a left photosensitive element (24) and a right photosensitive element (24) for performing video signal transmission for the naked eye 3D displays (3, 4) at the same time, wherein the first naked eye 3D display (3) and the second naked eye 3D display (4) are reversely connected to the photosensitive elements (24), wherein a left input port of the second naked eye 3D display (4) is connected with the right photosensitive element (24), wherein a right input port is connected with the left photosensitive element (24), wherein display directions of the first naked eye 3D display (3) and the second naked eye 3D display (4) are opposite, and directions of an image displayed by the first naked eye 3D display (3) and an image displayed by the second naked eye 3D display (4) are different by 180 degrees.

2. The surgical microscope having assistant's device according to claim 1, further comprising a mounting rack (5), wherein the first naked eye 3D display (3) and the second naked eye 3D display (4) are respectively movably mounted on the mounting rack (5).

3. The surgical microscope having assistant's device according to claim 2, wherein the first naked eye 3D display (3) and the second naked eye 3D display (4) are respectively able to be driven to independently move on the mounting rack (5) in a vertical direction.

4. The surgical microscope having assistant's device according to claim 2, wherein the support (1) comprises a base (11), a support rod (15) vertically mounted on the base (11), a large cross arm (12) rotatably mounted on the support rod (15), a small cross arm (13) rotatably mounted on the large cross arm (12) and a balance arm (14) rotatably mounted on the small cross arm (13), and the microscope body (2) is mounted on the balance arm (14).

5. The surgical microscope having assistant's device according to claim 4, wherein the first naked eye 3D display (3) and the second naked eye 3D display (4) are mounted on the microscope body (2), the large cross arm (12) or the support rod (15) through the mounting rack (5); or the surgical microscope having assistant's device further comprises a seat body (6) and a connecting rod (61) mounted on the seat body (6), and the first naked eye 3D display (3) and the second naked eye 3D display (4) are mounted on one end of the connecting rod (61) through the mounting rack (5).

6. The surgical microscope having assistant's device according to claim 5, wherein the other end of the connecting rod (61) is movably mounted on the seat body (6), the connecting rod (61) is able to be driven to move in an axis direction, and/or the connecting rod (61) is able to be driven to rotate by taking an axis of the connecting rod as a rotating shaft.

7. The surgical microscope having assistant's device according to claim 6, wherein the first naked eye 3D display (3) and the second naked eye 3D display (4) are able to be placed on the ground or suspended on a roof through the seat body (6) and the connecting rod (61).

8. The surgical microscope having assistant's device according to claim 1, wherein a size of the first naked eye 3D display (3) and the second naked eye 3D display (4) is between 12 inches and 16 inches; and
the microsurgery auxiliary device further comprises an acquisition device, a processing device and a driving device, the acquisition device is able to be configured to acquire eye position information of an operator (9) or an assistant (10), and the processing device is configured to be able to control the driving device to act according to the acquired eye position information to regulate a display angle of the first naked eye 3D display (3) or the second naked eye 3D display (4).

9. The surgical microscope having assistant's device according to claim 5, further comprising a plurality of wheels (62), wherein the wheels (62) are mounted at a bottom end of the base (11) or the seat body (6).

10. The surgical microscope having assistant's device according to claim 9, wherein the wheels (62) are universal wheels.

## Patentansprüche

1. Ein Operationsmikroskop mit Assistentenvorrichtung, umfassend eine Stütze (1), einen Mikroskopkörper (2), ein erstes brillenloses 3D-Display (3) und ein zweites brillenloses 3D-Display (4), wobei der Mikroskopkörper (2) an der Stütze (1) montiert ist, wobei zwei photosensitive Elemente (24) in dem Mikroskopkörper (2) angeordnet sind, wobei die zwei photosensitiven Elemente (24) jeweils als ein linkes photosensitives Element (24) und ein rechtes photosensitives Element (24) definiert sind, um gleichzeitig eine Videosignalübertragung für die brillenlosen 3D-Displays (3, 4) auszuführen, wobei das erste brillenlose 3D-Display (3) und das zweite brillenlose 3D-Display (4) in umgekehrter Weise mit den photosensitiven Elementen (24) verbunden sind, wobei ein linker Eingangsport des zweiten brillenlosen 3D-Displays (4) mit dem rechten photosensitiven Element (24) verbunden ist, wobei ein rechter Eingangsport mit dem linken photosensitiven Element (24) verbunden ist, wobei Anzeigerichtungen des ersten brillenlosen 3D-Displays (3) und des zweiten brillenlosen 3D-Displays (4) entgegengesetzt sind, und Richtungen eines durch das erste brillenlose 3D-Display (3) angezeigten Bildes und eines durch das zweite brillenlose 3D-Display (4) angezeigten Bildes um 180 Grad unterschiedlich sind.

2. Das Operationsmikroskop mit Assistentenvorrichtung nach Anspruch 1, ferner umfassend einen Montagerahmen (5), wobei das erste brillenlose 3D-Display (3) und das zweite brillenlose 3D-Display (4) jeweils beweglich an dem Montagerahmen (5) montiert sind.

3. Das Operationsmikroskop mit Assistentenvorrichtung nach Anspruch 2, wobei das erste brillenlose 3D-Display (3) und das zweite brillenlose 3D-Display (4) jeweils angetrieben werden können, sich unabhängig voneinander in vertikaler Richtung auf dem Montagerahmen (5) zu bewegen.

4. Das Operationsmikroskop mit Assistentenvorrichtung nach Anspruch 2, wobei die Stütze (1) eine Basis (11), eine auf der Basis (11) vertikal montierte Stützstange (15), einen auf der Stützstange (15) drehbar montierten großen Querarm (12), einen auf dem großen Querarm (12) drehbar montierten kleinen Querarm (13) und einen auf dem kleinen Querarm (13) drehbar montierten Balancearm (14) umfasst, und der Mikroskopkörper (2) auf dem Balancearm (14) montiert ist.

5. Das Operationsmikroskop mit Assistentenvorrichtung nach Anspruch 4, wobei das erste brillenlose 3D-Display (3) und das zweite brillenlose 3D-Display (4) über den Montagerahmen (5) an dem Mikroskopkörper (2), dem großen Querarm (12) oder der Stützstange (15) montiert sind; oder das Operationsmikroskop mit Assistentenvorrichtung ferner einen Standkörper (6) und eine auf dem Standkörper (6) montierte Verbindungsstange (61) umfasst, und das erste brillenlose 3D-Display (3) und das zweite brillenlose 3D-Display (4) an einem Ende der Verbindungsstange (61) über den Montagerahmen (5) montiert sind.

6. Das Operationsmikroskop mit Assistentenvorrichtung nach Anspruch 5, wobei das andere Ende der Verbindungsstange (61) beweglich an dem Standkörper (6) montiert ist, die Verbindungsstange (61) angetrieben werden kann, sich in Achsrichtung zu bewegen, und/oder die Verbindungsstange (61) angetrieben werden kann, um unter Verwendung einer Achse der Verbindungsstange als Drehachse zu rotieren.

7. Das Operationsmikroskop mit Assistentenvorrichtung nach Anspruch 6, wobei das erste brillenlose 3D-Display (3) und das zweite brillenlose 3D-Display (4) über den Standkörper (6) und die Verbindungsstange (61) auf dem Boden aufgestellt oder an einer Decke aufgehängt werden können.

8. Das Operationsmikroskop mit Assistentenvorrichtung nach Anspruch 1, wobei eine Größe des ersten brillenlosen 3D-Displays (3) und des zweiten brillenlosen 3D-Displays (4) zwischen 12 Zoll und 16 Zoll liegt; und
die mikrochirurgische Hilfsvorrichtung ferner eine Erfassungseinrichtung, eine Verarbeitungseinrichtung und eine Antriebseinrichtung umfasst, wobei die Erfassungseinrichtung dazu ausgebildet sein kann, Augenpositionsinformationen eines Operateurs (9) oder eines Assistenten (10) zu erfassen, und die Verarbeitungseinrichtung dazu ausgebildet ist, die Antriebseinrichtung entsprechend den erfassten Augenpositionsinformationen anzusteuern, um einen Anzeigewinkel des ersten brillenlosen 3D-Displays (3) oder des zweiten brillenlosen 3D-Displays (4) einzustellen.

9. Das Operationsmikroskop mit Assistentenvorrichtung nach Anspruch 5, ferner umfassend eine Mehrzahl von Rädern (62), wobei die Räder (62) an einem unteren Ende der Basis (11) oder des Standkörpers (6) montiert sind.

10. Das Operationsmikroskop mit Assistentenvorrichtung nach Anspruch 9, wobei die Räder (62) Lenkrollen sind.

## Revendications

1. Un microscope chirurgical doté d'un dispositif d'assistance, comprenant un support (1), un corps de microscope (2), un premier écran 3D sans lunettes (3) et un deuxième écran 3D sans lunettes (4), dans lequel le corps de microscope (2) est monté sur le support (1), deux éléments photosensibles (24) étant disposés dans le corps de microscope (2), les deux éléments photosensibles (24) étant respectivement définis comme un élément photosensible gauche (24) et un élément photosensible droit (24) pour réaliser simultanément une transmission de signal vidéo pour les écrans 3D sans lunettes (3, 4), dans lequel le premier écran 3D sans lunettes (3) et le deuxième écran 3D sans lunettes (4) sont reliés de manière inversée aux éléments photosensibles (24), dans lequel un port d'entrée gauche du deuxième écran 3D sans lunettes (4) est relié à l'élément photosensible droit (24), dans lequel un port d'entrée droit est relié à l'élément photosensible gauche (24), dans lequel les directions d'affichage du premier écran 3D sans lunettes (3) et du deuxième écran 3D sans lunettes (4) sont opposées, et les directions d'une image affichée par le premier écran 3D sans lunettes (3) et d'une image affichée par le deuxième écran 3D sans lunettes (4) diffèrent de 180 degrés.

2. Le microscope chirurgical doté d'un dispositif d'assistance selon la revendication 1, comprenant en outre un support de montage (5), dans lequel le premier écran 3D sans lunettes (3) et le deuxième écran 3D sans lunettes (4) sont respectivement montés de manière mobile sur le support de montage (5).

3. Le microscope chirurgical doté d'un dispositif d'assistance selon la revendication 2, dans lequel le premier écran 3D sans lunettes (3) et le deuxième écran 3D sans lunettes (4) peuvent respectivement être entraînés à se déplacer indépendamment sur le support de montage (5) selon une direction verticale.

4. Le microscope chirurgical doté d'un dispositif d'assistance selon la revendication 2, dans lequel le support (1) comprend une base (11), une tige de support (15) montée verticalement sur la base (11), un grand bras transversal (12) monté de manière rotative sur la tige de support (15), un petit bras transversal (13) monté de manière rotative sur le grand bras transversal (12) et un bras d'équilibrage (14) monté de manière rotative sur le petit bras transversal (13), et le corps de microscope (2) est monté sur le bras d'équilibrage (14).

5. Le microscope chirurgical doté d'un dispositif d'assistance selon la revendication 4, dans lequel le premier écran 3D sans lunettes (3) et le deuxième écran 3D sans lunettes (4) sont montés sur le corps de microscope (2), le grand bras transversal (12) ou la tige de support (15) par l'intermédiaire du support de montage (5) ; ou le microscope chirurgical doté d'un dispositif d'assistance comprend en outre un socle (6) et une tige de connexion (61) montée sur le socle (6), et le premier écran 3D sans lunettes (3) et le deuxième écran 3D sans lunettes (4) sont montés sur une extrémité de la tige de connexion (61) par l'intermédiaire du support de montage (5).

6. Le microscope chirurgical doté d'un dispositif d'assistance selon la revendication 5, dans lequel l'autre extrémité de la tige de connexion (61) est montée de manière mobile sur le socle (6), la tige de connexion (61) pouvant être entraînée à se déplacer selon une direction axiale, et/ou la tige de connexion (61) pouvant être entraînée à tourner en prenant l'axe de la tige de connexion comme axe de rotation.

7. Le microscope chirurgical doté d'un dispositif d'assistance selon la revendication 6, dans lequel le premier écran 3D sans lunettes (3) et le deuxième écran 3D sans lunettes (4) peuvent être posés au sol ou suspendus au plafond par l'intermédiaire du socle (6) et de la tige de connexion (61).

8. Le microscope chirurgical doté d'un dispositif d'assistance selon la revendication 1, dans lequel une taille du premier écran 3D sans lunettes (3) et du deuxième écran 3D sans lunettes (4) est comprise entre 12 pouces et 16 pouces ; et
le dispositif auxiliaire de microchirurgie comprend en outre un dispositif d'acquisition, un dispositif de traitement et un dispositif d'entraînement, le dispositif d'acquisition pouvant être configuré pour acquérir des informations de position des yeux d'un opérateur (9) ou d'un assistant (10), et le dispositif de traitement étant configuré pour commander le dispositif d'entraînement à agir selon les informations de position des yeux acquises afin de régler un angle d'affichage du premier écran 3D sans lunettes (3) ou du deuxième écran 3D sans lunettes (4).

9. Le microscope chirurgical doté d'un dispositif d'assistance selon la revendication 5, comprenant en outre une pluralité de roues (62), dans lequel les roues (62) sont montées à une extrémité inférieure de la base (11) ou du socle (6).

10. Le microscope chirurgical doté d'un dispositif d'assistance selon la revendication 9, dans lequel les roues (62) sont des roulettes universelles.
